# EUROPÄISCHE PATENTSCHRIFT

(11) **EP 2 440 512 B1**
(45) Veröffentlichungstag und Bekanntmachung des Hinweises auf die Patenterteilung: **21.01.2015**
(21) Anmeldenummer: 10724417.0
(22) Anmeldetag: 31.05.2010
(51) Int. Cl.: C07C 51/47, C07C 51/50, C08K 5/13

(54) **VERFAHREN ZUR UMSTABILISIERUNG VON (METH)ACRYLMONOMEREN**
PROCESS FOR RESTABILISATION OF (METH)ACRYLIC MONOMERS
PROCÉDÉ POUR LA RÉSTABILISATION DES MONOMÈRES (METH)ACRLYLIQUES

(30) Priorität: 08.06.2009 DE 102009026822; 08.06.2009 US 184865 P
(43) Veröffentlichungstag der Anmeldung: 18.04.2012
(73) Patentinhaber: BASF SE, 67056 Ludwigshafen (DE)
(72) Erfinder: HERFERT, Norbert, 63674 Altenstadt (DE); HAMMON, Ulrich, 68163 Mannheim (DE); WÜSTEFELD, Renate, 67346 Speyer (DE)
(86) Internationale Anmeldenummer: PCT/EP2010/057519
(87) Internationale Veröffentlichungsnummer: WO 2010/142546

(56) Entgegenhaltungen:
- EP-A1- 0 179 476
- EP-A1- 0 775 686
- WO-A2-2004/052819

## Beschreibung

Die vorliegende Erfindung betrifft ein Verfahren zur Umstabilisierung von (Meth)acrylmonomeren.

Unter dem Begriff (Meth)acrylmonomere im Sinne der vorliegenden Erfindung werden Substanzen verstanden, die aus Acrolein, Methacrolein, Acrylsäure, Methacrylsäure und/oder aus Estern der beiden vorgenannten Säuren bestehen. (Meth)acryl wird in dieser Schrift generell als verkürzte Schreibweise für Acryl und/oder Methacryl verwendet.

(Meth)acrylmonomere sind ethylenisch ungesättigte Verbindungen, die radikalisch polymerisieren können. Einmal ausgelöst, verlaufen die radikalischen Polymerisationen normalerweise ausgeprägt exotherm, d.h. unter starker Wärmeentwicklung, wobei die freigesetzte Polymerisationswärme, für den Fall, dass die sie nicht abgeführt wird, die radikalische Polymerisation zusätzlich beschleunigt.

Erfolgt bei gewollten radikalischen Polymerisationen vorgenannte Wärmeabfuhr in mangelhafter Weise, besteht daher die Gefahr, dass die Polymerisation so heftig verläuft, dass das das Polymerisationsgemisch enthaltende Gefäß explodiert, wenn der außer Kontrolle geratenen Polymerisation nicht entgegen getreten wird.

Ein solches wirksames Entgegentreten wird insbesondere im Fall von ungewollt ausgelösten radikalischen Polymerisationen benötigt. Ungewollt ausgelöste radikalische Polymerisationen können z.B. bei der Lagerung und/oder beim Transport von Monomere enthaltenden Substanzen auftreten, da sowohl Wärme als auch Licht oder unerwünschte Radikale eine radikalische Polymerisation von Monomeren auslösen können. Zwar versucht man solchen ungewollten radikalischen Polymerisationen üblicherweise dadurch präventiv entgegenzutreten, dass man den Monomeren geringe Menge (in der Regel bis zu 1000 ppm) an Polymerisationsinhibitoren zusetzt. Allerdings darf die inhibierende Wirkung nicht zu ausgeprägt sein, da die Monomere für ihre spätere Verwendung einer gewollten radikalischen Polymerisation unterworfen werden müssen. Eine mäßig inhibierende Wirkung, wie sie z.B. Hydrochinonmonomethylether (MEHQ) aufweist, kann von radikalischen Polymerisationsinitiatoren jedoch normalerweise dominiert werden, weshalb MEHQ ein für Monomere besonders häufiger Lager- und/oder Transportstabilisator ist. Die Erfahrung hat jedoch gezeigt, dass selbst bei mit Lager- und/oder Transportstabilisatoren stabilisierten Monomeren eine ungewollte radikalische Polymerisation derselben nicht völlig ausgeschlossen werden kann. Dies gilt insbesondere für die besonders polymerisationsfreudigen (Meth)acrylmonomere, insbesondere für (Meth)acrylsäure.

Die Polymerisationsneigung von (Meth)acrylmonomeren ist besonders dann gegeben, wenn solche Substanzen beim Transport und/oder bei der Lagerung extremen äußeren Bedingungen augesetzt sind (z.B. extrem hohe Temperaturen beim Transport via Schiff über verschiedene Klimazonen hinweg, wie es z.B. beim Transport von Europa nach Südostasien oder Südamerika der Fall ist).

Für einen sicheren Transport von (Meth)acrylmonomeren werden diese üblicherweise mit Phenothiazin (PTZ) oder Gemischen von PTZ mit anderen Polymerisationsinhibitoren stabilisiert. PTZ ist ein äußerst wirksamer Stabilisator für (Meth)acrylmonomere. Eine Weiterverarbeitung der PTZ-haltigen (Meth)acrylmonomere ist jedoch nahezu unmöglich, da eine Polymerisation in Gegenwart von PTZ unterbunden ist. Daher müssen PTZ-haltige (Meth)acrylmonomere vor einer Weiterverarbeitung aufgereinigt werden, in dem PTZ vollständig entfernt wird. Diese Entfernung erfolgt üblicherweise durch Destillation oder Kristallisation, wie beispielsweise in WO 05/007610 A1 und in WO 02/090299 A2 beschrieben.

Nachteilig daran ist, dass die Abtrennung des PTZ von den (Meth)acrylmonomeren durch Destillation und Kristallisation sehr aufwendig ist, insbesondere weil die (Meth)acrylmonomere zunächst in den gasförmigen oder festen Aggregatzustand überführt werden müssen, um das PTZ vollständig entfernen zu können. Anschließend muss ein anderer, mäßig inhibierender Stabilisator wie beispielsweise MEHO zugegeben werden, damit eine ungewollte radikalische Polymerisation noch vor der geplanten Weiterverarbeitung vermieden wird.

Aus EP 0 775 686 A1 ist ein Verfahren zur adsorptiven Trennung von PTZ aus Acrylsäure bekannt. Demgemäß wird eine PTZ-haltige Acrylsäurelösung mit einem Bentonit in Kontakt gebracht, um den PTZ-Gehalt auf unter 100 ppm zu reduzieren. Nachteilig an diesem Verfahren ist, dass der PTZ-Gehalt nach Adsorption immer noch zu hoch ist, um die Acrylsäure einer weiteren Verarbeitung zuzuführen.

Aufgabe der vorliegenden Erfindung war es daher, ein Verfahren zur Umstabilisierung von (Meth)acrylmonomeren zur Verfügung zu stellen, bei dem Phenothiazin aus Phenothiazin-haltigen (Meth)acrylmonomeren effektiv und einfach entfernt wird und 'falls erforderlich, anschließend ein mäßig wirksamer Polymerisationsinhibitor, der die Weiterverarbeitung nicht wesentlich behindert, zugesetzt wird.

Gelöst wurde diese Aufgabe durch ein Verfahren zur Umstabilisierung von (Meth)acrylmonomeren, wobei aus Phenothiazin-haltigen (Meth)acrylmonomeren Phenothiazin durch Adsorption an Aktivkohle entfernt wird und gegebenenfalls anschließend ein mäßig wirksamer Polymerisationsinhibitor zugegeben wird.

Vorteilhaft an dem erfindungsgemäßen Verfahren ist, dass die adsorptive Abtrennung des PTZ einfach und nahezu vollständig erfolgt. Besonders vorteilhaft ist, dass die (Meth)acrylmonomere im flüssigen Aggregatzustand, wie sie beispielsweise beim Transport vorliegen, direkt der Adsorption zugeführt werden können.

Üblicherweise enthalten (Meth)acrylmonomere bis zu 1000 ppm, bevorzugt bis zu 500 ppm und insbesondere um 200 ppm Phenothiazin als Polymerisationsinhibitor. Derartig stabilisierte (Meth)acrylmonomere können gefahrlos auch über weite Distanzen transportiert werden.

Für die erfindungsgemäße Reinigung von (Meth)acrylmonomeren durch Adsorption an Aktivkohle können Adsorptionsverfahren eingesetzt werden, wie sie beispielsweise in Ullmann's Enzyklopädie der Technischen Chemie, 4. Auflage, Vol. 2, S. 600-619 und in der dort zitierten Literatur beschrieben und dem Fachmann bekannt sind. Hierbei handelt es sich beispielsweise um Festbettverfahren, bei denen die Aktivkohle fest angeordnet und das zu reinigende (Meth)acrylmonomer in geflutetem Zustand bzw. in der Rieselphase abwärts oder aber von unten nach oben durchströmt wird.

Von der Bauweise des Festbettadsorber her unterscheidet man z.B. Horizontal-oder Vertikaladsorber und einfache Adsorptionsbecken. Andere Adsorptionsverfahren arbeiten stattdessen mit bewegten Adsorptionsmittel, beispielsweise in der Wanderschicht oder in der Wirbelschicht. Bei Einsatz von pulverförmigen Aktivkohlen unterscheidet man weiterhin Einrührverfahren, bei denen das Adsorptionsmittel mit dem zu reinigenden (Meth)acrylmonomer in einem Rührbehälter verrührt und anschließend in einer Filterpresse abfiltriert wird, und das Verfahren der Schichtfiltration, bei dem zunächst durch Aufschwemmen auf dem Filter eine Adsorptionsmittelschicht erzeugt wird, durch die das zu reinigende (Meth)acrylmonomer hindurchgedrückt wird.

Für die erfindungsgemäße Reinigung von (Meth)acrylmonomeren durch Adsorption an Aktivkohle werden bevorzugt Festbettverfahren herangezogen. In vorteilhafter Weise werden zwei Adsorptionsbetten vorgesehen, um bei Erschöpfung des einen Adsorptionsbettes auf das andere umschalten und die verbrauchte Aktivkohle regenerieren bzw. ersetzen zu können. Denkbar ist daher sowohl eine Parallelschaltung von zwei Adsorptionsbetten als auch bevorzugt eine Anordnung von zwei Adsorptionsbetten in Reihe. Besonders bevorzugt ist der Einsatz von vier Adsorptionsbetten in Form einer Parallelanordnung von jeweils zwei Adsorptionsbetten in Reihe. Durch diese besonders bevorzugte Ausführungsform kann gewährleistet werden, dass bei Erschöpfung der ersten Adsoptionsbetten die jeweils in Reihe geschalteten zweiten Adsorptionsbetten restliches PTZ aus den (Meth)acrylmonomeren ausreichend entfernen. Je nach gewähltem Verfahren muss der erfindungsgemäßen Reinigung des (Meth)acrylmonomeren durch Adsorption ein Filtrationsschritt nachgeschaltet werden. Die bei der erfindungsgemäßen Reinigung des (Meth)acrylmonomeren durch Adsorption einzustellenden Parameter, wie Temperatur, Druck, Verweilzeit, sind von der Wahl des Adsorptionsverfahrens abhängig und beeinflussen in einer dem Fachmann bekannten Weise das Ergebnis der Reinigung. Beispielsweise muss für die Reinigung des (Meth)acrylmonomeren eine Temperatur oberhalb des Schmelzpunktes des jeweiligen (Meth)acrylmonomeren eingehalten werden. Maximal möglich ist die Siedetemperatur des jeweiligen (Meth)acrylmonomeren. Im Falle von Acrylsäure liegt der Schmelzpunkt unter Normalbedingungen bei 14°C, die Siedetemperatur bei 141°C. Die Schmelz- und Siedepunkte der jeweiligen zu reinigenden (Meth)acrylmonomere variieren selbstverständlich mit deren Reinheitsgrad.

Prinzipiell sind als Adsorptionstemperatur also 15 bis 140°C, bevorzugt 20 bis 100°C und besonders bevorzugt 20 bis 80°C einzustellen. Besonders bevorzugt wird die Adsorption bei Umgebungstemperatur durchgeführt. Der Druck sollt im Bereich 1 bis 100 bar, bevorzugt 1 bis 10 bar, besonders bevorzugt 1 bis 5 bar liegen.

Unter Aktivkohle im Sinne der vorliegenden Erfindung ist aktivierter Kohlenstoff zu verstehen, der aus unterschledlichen, Kohlenstoff liefernden Vorprodukten hergestellt werden kann. Die Verfahren zur Überführung in die aktive Form können ebenfalls sehr unterschiedlich sein. Bei solchen Herstellungsverfahren werden Aktivkohlen erhalten, die BET-Oberflächen von 200 bis 3000 m²/g, bevorzugt 300 bis 2000 m²/g, besonders bevorzugt 500 bis 1800 m²/g und Schüttgewichte zwischen 250 und 550 g/l aufweisen.

Als Ausgangsmaterialien zur Herstellung aktivierter Kohlen seien beispielsweise genannt: Sägemehl und andere Holzabfalle, Stroh, verschiedene Kohlensorten, wie Bitumen- oder Braunkohle, Nussschalen wie beispielweise Kokosnuss, Mineralölteere, Lignin, Polysaccharide, Polyacrylnitril, Knochen oder Torf. Ferner können auch Koksprodukte aus Braun- und Steinkohlen eingesetzt werden. In bevorzugter Weise seien genannt: Holz, Cellulose, Lignin, Bitumen- oder Braunkohle, Nussschalen, Torf oder Steinkohlenkoks.

Die genannten Kohlenstoff liefernden Vorprodukte können durch verschiedene Methoden aktiviert werden, beispielsweise durch chemische Aktivierung mit Phosphorsäure oder Zinkchlorid, durch Gasaktivierung mit Dampf, Sauerstoff oder Nitrose enthaltenden Gase. Solche voraktivierten Vorprodukte werden sodann thermisch, d.h. durch Verkoken in Aktivkohlen für das erfindungsgemäße Verfahren übergeführt. Diese Herstellungsweisen sind dem Fachmann bekannt und sind genau wie die nähere Beschreibund der dabei erhältlichen verschiedenen Sorten von Aktivkohle in der Literatur ausführlich beschrieben (s. Ullmann's Encyclopedia of Industrial Chemistry, 5th Ed., Vol. A5 (1986), S. 124-140 und die dort zitierte Literatur).

Hinsichtlich der Anwendungsform können in das erfindungsgemäße Verfahren Formkohlen, Kornkohlen und Pulverkohlen eingesetzt werden. Bei Formkohlen, die meist durch Strangpressen aus Pulvern hergestellt werden und dann zylindrische Form aufweisen oder seltener als Pellets vorliegen, kommen meist Durchmesser im Bereich von ein bis einigen mm in Frage. Bei Pulverkohlen muss besonders auf ausreichende Filtrierbarkeit geachtet werden.

Dem Fachmann ist die von der Wahl des Adsorptionsverfahrens abhängige optimale Anwendungsform der Aktivkohle bekannt.

Eine Regenerierung der Aktivkohle ist prinzipiell möglich und kann in Abhängigkeit von der Wirtschaftlichkeit auch durchgeführt werden. Die adsorbierten Salze und farbgebenden Verbindungen können gegebenenfalls mit Wasser, mit Methanol, Methanol/Wasser, mit Glykol oder mit Glykol-Wasser-Gemischen von der Aktivkohle abgewaschen und auf diese Weise eine Regenerierung erreicht werden. Bei kontinuierlicher Arbeitsweise kann die eingesetzte Aktivkohle über lange Zeit im Adsorber verbleiben. Unlösliche organische Ablagerungen können durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800 °C oder durch Überleiten von 0,01 bis 5 Gew.-% Sauerstoff enthaltenden Verdünnungsgasen wie Stickstoff, Kohlenmonoxid oder Kohlendioxid oder durch Kohlendioxid allein bei 200 bis 800 °C entfernt werden. Die bevorzugte Regenerierungstemperatur liegt bei 250 bis 700 °C, besonders bevorzugt bei 250 bis 600 °C.

Für das erfindungsgemäße Verfahren eignen sich insbesondere käuflich erhältliche Aktivkohlen wie beispielsweise CPG^{®} LF der Firma Chemviron Carbon (BET 950-1050 m²/g), CAL^{®} der Firma Chemviron Carbon (BET 1050 m²/g), Epibon^{®} Y 12 x 40der Firma Donau Carbon (BET 1000 m²/g), Norit^{®} GAC 1240 N der Firma Norit (BET 1125 m²/g), Norit^{®} ROX 0,8 Supra der Firma Norit (BET 1225 m²/g), Acticarbone^{®} BGX der Firma Ceca Chemicals (keine Angabe zur BET-Oberfläche), Aktivkohle, pulv. der Firma Carl Roth (keine Angabe zur BET-Oberfläche), Norit^{®} CAP Super der Firma Norit (BET 1800 m²/g) und Norit^{®} CA1 der Firma Norit (BET 1400 m²/g).

Durch Adsorption gereinigten (Meth)acrylmonomere sind nahezu frei von PTZ, d.h. der PTZ-Gehalt nach Adsorption liegt im Regelfall unter 10 ppm, bezogen auf die (Meth)acrylmonomeren, bevorzugt unter 5 ppm, besonders bevorzugt unter 3 ppm, jeweils bezogen auf die (Meth)acrylmonomere.

Falls erforderlich, kann anschließend ein anderer, mäßig wirksamer Polymerisationsinhibitor zugesetzt werden, damit die (Meth)acrylmonomere vor einer Weiterverarbeitung nicht einer ungewollten radikalischen Polymerisation ausgesetzt sind. Der mäßig wirksame Polymerisationsinhibitor kann sowohl vor der adsorbierenden Behandlung als auch während der adsorbierenden Behandlung zugesetzt werden, wenn der mäßig wirksame Polymerisationsinhibitor nicht bzw. geringfügig während der adsorbtiven Behandlung adsorbiert wird. Wird der mäßig wirksame Polymerisationsinhibitor in erheblichen Umfang adsorbiert wird er günstigerweise direkt nach der Behandlung zugesetzt. Beispielsweise kann dieser mäßig wirksame Polymerisationsinhibitor in den üblichen Mengen zugesetzt werden, so dass der Inhibitor-Gehalt bis zu 1000 ppm, bevorzugt bis zu 500 ppm, besonders bevorzugt bis zu 200 ppm, ganz besonders bevorzugt bis zu 100 ppm und insbesondere um 50 ppm, jeweils bezogen auf die (Meth)acrylmonomere, beträgt.

Es ist ebenfalls möglich, dass ein Zusatz von mäßig wirksamem Polymerisationsinhibitor nicht erforderlich ist, insbesondere dann, wenn die (Meth)acrylmonomere bereits ausreichend mit einem solchen Inhibitor stabilisiert sind, um eine ungewollte radikalische Polymerisation vor der Weiterverarbeitung zu vermeiden. Eine solche ausreichende Stabilisierung liegt vor, wenn der Inhibitor-Gehalt bereits bis zu 250 ppm, bevorzugt höchstens 130 ppm, besonders bevorzugt höchstens 70 ppm, bevorzugt mindestens 10 ppm, besonders bevorzugt mindestens 30 ppm, insbesondere um 50 ppm, mäßig wirksamer Polymerisationsinhibitor, jeweils bezogen auf das (Meth)acrylmonomere, beträgt.

Ein mäßig wirksamer Polymerisationsinhibitor im Sinne der vorliegenden Erfindung ist ausgewählt aus der Gruppe der phenolischen Verbindungen und Chinone.

Unter phenolischen Verbindungen, die ggf. eine oder mehrere Alkylgruppen aufweisen, wie z.B. Alkylphenole, werden beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol verstanden.

Chinone eignen sich ebenfalls als mäßig wirksame Polymerisationsinhibitoren und sind beispielsweise Hydrochinon, Hydrochinonmonomethylether, 2-Methylhydrochinon, 2,5-Di-tert.-Butylhydrochinon oder Benzochinon.

Unter den zuvor genannten mäßig wirksamen Polymerisationsinhibitoren sind Chinone bevorzugt, besonders bevorzugt sind Hydrochinon und Hydrochinonmonomethylether (MEHQ), insbesondere bevorzugt ist MEHQ.

Überraschenderweise wurde festgestellt, dass mit dem erfindungsgemäßen Verfahren besonders effektiv und selektiv PTZ entfernt wird, gegebenenfalls ebenfalls vorhandener mäßig wirksamer Polymerisationsinhibitor wie MEHQ jedoch in größeren Mengen im (Meth)acrylmonomeren verbleibt. Dies ist insbesondere im Hinblick auf

WO 03/051940 A1, WO 2004/052819 A2 sowie JP 48-43331 überraschend. Gemäß der Lehre der WO 03/051940A1 wird MEHO bei unneutralisierter Acrylsäure durch die adsorptive Trennung an Aktivkohle auf einen Gehalt von 10 bis 160 ppm reduziert. WO 04/0552819 A2 sowie JP 48-43331 offenbaren die adsorptive Entfernung von MEHQ an Aktivkohle bei teil- bis vollneutralisierter Acrylsäure. Die vorliegende Erfindung zeigt jedoch, dass PTZ wesentlich effektiver entfernt wird als beispielsweise MEHQ.

Das erfindungsgemäße Verfahren eignet sich insbesondere für (Meth)acrylsäure und besonders bevorzugt für Acrylsäure. Acrylsäure wird in großen Mengen gelagert und weltweit transportiert, beispielsweise um in anderen Ländern superabsorbierende Polymere daraus herzustellen. Die Herstellung von Acrylsäure ist an sich bekannt und wird beispielsweise in WO 02/090299 A2, WO 05/007610 A1 und WO 06/092410 A1 beschrieben.

Für die Herstellung von superabsorbierenden Polymeren wird beispielsweise eine Acrylsäure mit folgender Spezifikation eingesetzt: 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Für die Herstellung von superabsorbierenden Polymeren werden neben der genannten Acrylsäure noch geeignete Vernetzer, Initiatoren sowie gegebenenfalls copolymerisierbare ethylenisch ungesättigte Monomere zugesetzt. Derartige Verbindungen sowie die Herstellung von superabsorbierenden Polymeren sind dem Fachmann beispielsweise aus Ullmanns's Encyclopedia of Industrial Chemistry. 6th Ed., Viley VCH, 2003 Vol. 35, "Superabsorbents", p. 73-93, bekannt.

Üblicherweise wird für die Herstellung der superabsorbierenden Polymere eine wässrige Acrylsäurelösung verwendet. Der Wassergehalt der Acrylsäurelösung beträgt vorzugsweise von 40 bis 75 Gew.-%, besonders bevorzugt von 45 bis 70 Gew.-%, ganz besonders bevorzugt von 50 bis 65 Gew.-%. Daher eignet sich das erfindungsgemäße Verfahren auch für Acrylsäurelösungen mit einem entsprechenden Wassergehalt von 40 bis 75 Gew.-%.

Bevorzugt wird das erfindungsgemäße Verfahren jedoch an Acrylsäurelösungen durchgeführt, denen noch kein Wasser zugesetzt wurde und die somit wasserfrei sind. Wasserfrei bedeutet, dass der Wassergehalt im Rahmen der üblichen, zuvor beschriebenen, Spezifikation der Acrylsäure liegt, d.h. der Wassergehalt beträgt weniger als 1 Gew.-%, bevorzugt weniger als 0,5 Gew.-% und besonders bevorzugt weniger als 0,05 Gew.-%.

Darüber hinaus ist es üblich, dass die Säuregruppen der erhaltenen superabsorbierenden Polymere teilweise neutralisiert sind. Die Neutralisation wird vorzugsweise auf der Stufe der Acrylsäuremonomere durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen.

In einer Ausführungsform des erfindungsgemäßen Verfahrens eignet sich dieses daher auch für Acrylsäuremonomerlösungen, deren Säuregruppen teilweise, beispielsweise im Bereich von 25 bis 95 mol-%, bevorzugt von 30 bis 80 mol-%, ganz besonders bevorzugt von 40 bis 75 mol-%, mit den zuvor genannten Neutralisationsmitteln neutralisiert sind.

In einer andern Ausführungsform ist es selbstverständlich auch möglich, unneutralisierte Acrylsäuremonomerlösungen durch adsorptive Trennung an Aktivkohle von PTZ zu reinigen.

### Beispiele

Die folgenden Beispiele sollen die Eigenschaften der Erfindung erläutern, ohne sie aber einzuschränken.

Falls nicht anders angegeben, bedeuten Prozent immer Gewichtsprozent und Teile immer Gewichtsteile.

### Monomerlösung 1: Herstellung einer acrylsäurehaltigen Lösung mit einem Neutralisationsgrad von 72 mol-% und einem Feststoffgehalt von 41 Gew.-%

In einem Edelstahlbecher wurden 80 Gewichtsteile 50 Gew.-%ige Natronlauge und 117,6 Gewichtsteile gefrorenes, entionisiertes Wasser vorgelegt. Unter Rühren wurden 100 Gewichtsteile Acrylsäure, die 317 ppm Phenothiazin (PTZ) und 100 ppm Hydrochinonmonomethylether (MEHQ) enthielt, zugegeben. Dabei wurde die Geschwindigkeit der Zugabe so eingestellt, dass die Temperatur in der Mischung 35 °C nicht überstieg. Während der Neutralisationsreaktion bildete sich ein Niederschlag, der über einen Papierfaltenfilter abfiltriert wurde. Die filtrierte Monomerlösung wurde mittels HPLC analysiert, sie enthielt 27 ppm PTZ und 29 ppm MEHQ.

### Monomerlösung 2: Herstellung einer acrylsäurehaltigen Lösung mit einem Neutralisationsgrad von 50 mol-% und einem Feststoffgehalt von 47 Gew.-%

In einem Edelstahlbecher wurden 55,56 Gewichtsteile 50 Gew.-%ige Natronlauge und 89,72 Gewichtsteile gefrorenes, entionisiertes Wasser vorgelegt. Unter Rühren wurden 100 Gewichtsteile Acrylsäure, die 317 ppm Phenothiazin (PTZ) und 100 ppm Hydrochinonmonomethylether (MEHQ) enthielt, zugegeben. Dabei wurde die Geschwindigkeit der Zugabe so eingestellt, dass die Temperatur in der Mischung 35 °C nicht überstieg. Während der Neutralisationsreaktion bildete sich ein Niederschlag, der über einen Papierfaltenfilter abfiltriert wurde. Die filtrierte Monomerlösung wurde mittels HPLC analysiert, sie enthielt 70 ppm PTZ und 38 ppm MEHQ.

### Beispiel 1

### Adsorption der PTZ-haltigen Monomerlösungen 1 und 2 mit Aktivkohle

Für das Beispiel 1 wurden sechs verschiedene, käuflich erhältliche Aktivkohlen eingesetzt. Im Einzelnen sind dies:
- Aktivkohle 1:: CPG^{®} LF der Firma Chemviron Carbon (BET 950-1050 m²/g)
- Aktivkohle 2:: CAL^{®} der Firma Chemviron Carbon (BET 1050 m²/g)
- Aktivkohle 3:: Epibon^{®} Y 12 x 40der Firma Donau Carbon (BET 1000 m²/g)
- Aktivkohle 4:: Norit^{®} GAC 1240 N der Firma Norit (BET 1125 m²/g)
- Aktivkohle 5:: Norit^{®} ROX 0,8 Supra der Firma Norit (BET 1225 m²/g)
- Aktivkohle 6:: Acticarbone^{®} BGX der Firma Ceca Chemicals (keine Angabe zur BET Oberfläche)

Die jeweiligen eingesetzten Aktivkohlen wurden über Nacht im Trockenschrank bei 120 °C getrocknet. Zu jeweils 150 g Monomerlösung wurden je ca. 0,15 g, 0,75 g und 1,5 g Aktivkohle gegeben und im temperierbaren Schüttelschrank bei 20 °C für ca. 18 h geschüttelt. Die Aktivkohle wurde anschließend mit Hilfe einer Filtration über einen Blaubandfilter abgetrennt. Die filtrierten Lösungen wurden mittels HPLC auf ihren Gehalt an PTZ und MEHQ analysiert, die Ergebnisse sind in den Tabellen 1 (Monomerlösung 1) und 2 (Monomerlösung 2) zusammengefasst.

**Tabelle 1**

| Ergebnisse der PTZ-haltigen Monomerlösung 1 nach Adsorption mit Aktivkohle | | | | | |
|---|---|---|---|---|---|
| Einwaage [g] Monomerlösung 1 | Aktivkohle | Einwaage [g] Aktivkohle | Farbe nach Filtration | MEHQ [ppm] | PTZ [ppm] |
| 150,260 | 1 | 0,1571 | rosa | 35 | 2 |
| 150,428 | 1 | 0,7613 | gering rosa | 24 | < 1 |
| 150,308 | 1 | 1,5204 | farblos | 16 | < 1 |
| 151,228 | 2 | 0,1578 | rosa | 36 | 2 |
| 153,223 | 2 | 0,7598 | fast farblos | 30 | < 1 |
| 151,111 | 2 | 1,5221 | farblos | 25 | < 1 |
| 150,579 | 3 | 0,1522 | rosa | 36 | 3 |
| 150,119 | 3 | 0,7563 | gering rosa | 26 | < 1 |
| 150,556 | 3 | 1,5108 | farblos | 18 | < 1 |
| 151,035 | 4 | 0,1520 | rosa | 37 | 3 |
| 150,589 | 4 | 0,7558 | gering rosa | 31 | < 1 |
| 151,451 | 4 | 1,5061 | gering rosa | 27 | < 1 |
| 150,937 | 5 | 0,1507 | rosa | 34 | 2 |
| 150,554 | 5 | 0,7519 | gering rosa | 17 | < 1 |
| 150,313 | 5 | 1,5089 | farblos | 8 | < 1 |
| 150,615 | 6 | 0,1520 | rosa | 37 | 2 |
| 151,968 | 6 | 0,7554 | gering rosa | 32 | < 1 |
| 150,671 | 6 | 1,5008 | farblos | 29 | < 1 |

**Tabelle 2**

| Ergebnisse der PTZ-haltigen Monomerlösung 2 nach Adsorption mit Aktivkohle | | | | | |
|---|---|---|---|---|---|
| Einwaage [g] Monomerlösung 1 | Aktivkohle | Einwaage [g] Aktivkohle | Farbe nach Filtration | MEHQ [ppm] | PTZ [ppm] |
| 152,796 | 1 | 0,1505 | rosa | 26 | < 1 |
| 150,456 | 1 | 0,7529 | gering rosa | 17 | < 1 |
| 150,878 | 1 | 1,5161 | farblos | 11 | < 1 |
| 154,246 | 2 | 0,1562 | gering rosa | 26 | < 1 |
| 151,007 | 2 | 0,7588 | fast farblos | 18 | < 1 |
| 150,829 | 2 | 1,5089 | farblos | 11 | < 1 |
| 150,928 | 3 | 0,1570 | rosa | 25 | < 1 |
| 153,776 | 3 | 0,7564 | gering rosa | 16 | < 1 |
| 150,606 | 3 | 1,5041 | farblos | 11 | < 1 |
| 151,201 | 4 | 0,1524 | rosa | 27 | < 1 |
| 150,318 | 4 | 0,7589 | gering rosa | 22 | < 1 |
| 150,415 | 4 | 1,5137 | gering rosa | 17 | < 1 |
| 150,832 | 5 | 0,1559 | rosa | 23 | < 1 |
| 150,074 | 5 | 0,7520 | gering rosa | 11 | < 1 |
| 151,192 | 5 | 1,5047 | farblos | 5 | < 1 |
| 150,450 | 6 | 0,1611 | gering rosa | 26 | < 1 |
| 150,789 | 6 | 0,7604 | gering rosa | 21 | < 1 |
| 151,723 | 6 | 1,5212 | farblos | 16 | < 1 |

### Beispiel 2

### Adsorption von PTZ-haltigen nicht neutralisierter Acrylsäurelösungen (0 Gew.-% bzw. 20 Gew.-% Wassergehalt)

Für das Beispiel 2 wurden zwei verschiedene, käuflich erhältliche Aktivkohlen eingesetzt. Im Einzelnen sind dies:
- Aktivkohle 7:: Aktivkohle, pulv. der Firma Carl Roth (keine Angabe zur BET-Oberfläche)
- Aktivkohle 8:: Norit^{®} CAP Super der Firma Norit (BET 1800 m²/g)

In einem 50 mL Penicillinglas wurden jeweils 20,0 g Acrylsäure eingewogen. Die Acrylsäurelösungen mit 0 Gew.-% Wassergehalt enthielten 239 ppm PTZ zur Stabilisierung, die mit 20 Gew.-% Wasseranteil 196 ppm PTZ. Die Lösungen wurden auf einem Magnetrührer bei 400 Upm gerührt. Anschließend wurden 5 Gew.-% einer Aktivkohle, bezogen auf den Acrylsäuregehalt, zugegeben. Die Mischungen wurde n3 h bei Raumtemperatur gerührt. Anschließend wurden jeweils Proben entnommen, die zunächst über einen Spritzenfilter filtriert wurden und dann mittels HPLC auf ihren PTZ-Gehalt analysiert wurden. Die Ergebnisse sind in Tabelle 3 zusammengefasst.

### Vergleichsbeispiel 1

Beispiel 2 wurde wiederholt, allerdings waren die Acrylsäurelösungen mit 177 ppm (0 Gew.-% Wassergehalt) bzw. 140 ppm (20 Gew.-% Wassergehalt) MEHQ stabilisiert. Die Ergebnisse sind in Tabelle 3 zusammengefasst.

**Tabelle 3**

| Ergebnisse der PTZ-bzw. MEHQ-haltigen Acrylsäurelösungen nach Adsorption mit Aktivkohle | | | | |
|---|---|---|---|---|
| Beispiel 2 (PTZ-haltig) | Wassergehalt [Gew.-%] | Aktivkohle | PTZ bzw. MEHQ [ppm] | % PTZ bzw. MEHQ entfernt |
| | 0 | 7 | 4 | 98,3 |
| | 20 | 7 | 2 | 99,0 |
| | 0 | 8 | 0 | 100,0 |
| Vergleichsbeispiel 1 (MEHQ-haltig) | 0 | 7 | 146 | 17,5 |
| | 20 | 7 | 124 | 11,4 |
| | 0 | 8 | 130 | 26,6 |

### Beispiel 3

### Adsorption von PTZ-haltigen teilneutralisierten Acrylsäurelösungen (60 Gew.-% Wassergehalt)

Für das Beispiel 3 wurden drei verschiedene, käuflich erhältliche Aktivkohlen eingesetzt. Im Einzelnen sind dies:
- Aktivkohle 7:: Aktivkohle, pulv. der Firma Carl Roth (keine Angabe zur BET-Oberfläche)
- Aktivkohle 8:: Norit^{®} CAP Super der Firma Norit (BET 1800 m²/g)
- Aktivkohle 9:: Norit^{®} CA1 der Firma Norit (BET 1400 m²/g)

Zu jeweils 100,0 g einer 99,6 Gew.-%igen Acrylsäurelösung, die mit 185 ppm PTZ stabilisert war, wurden 79,61 g einer 50 Gew.-%igen Natronlauge gegeben (Neutralisationsgrad 72 mol-%). Anschließend wurden 169,90 g Wasser zugegeben, so dass der Wassergehalt der resultierenden Lösung 60 Gew.-% betrug. Es bildete sich ein feiner Niederschlag, der abfiltriert wurde. Den filtrierten Lösungen wurden jeweils 0,5 Gew.-% einer Aktivkohle, bezogen auf den Acrylsäuregehalt, zugegeben. Die Mischungen wurden 3 h bei Raumtemperatur gerührt. Anschließend wurde Proben entnommen, die zunächst über einen Spritzenfilter filtriert wurden und dann mittels HPLC auf ihren PTZ-Gehalt analysiert wurden.

| | |
|---|---|
| PTZ-Gehalt vor Neutralisation: | 185 ppm |
| PTZ-Gehalt nach Filtration, vor Zugabe der Aktivkohlen | 21 ppm |
| PTZ-Gehalt (Aktivkohle 7): | << 1 ppm |
| PTZ-Gehalt (Aktivkohle 8): | << 1 ppm |
| PTZ-Gehalt (Aktivkohle 9): | << 1 ppm |

## Patentansprüche

1. Verfahren zur Umstabilisierung von (Meth)acrylmonomeren, **dadurch gekennzeichnet, dass** aus Phenothiazin-haltigen (Meth)acrylmonomeren Phenothiazin durch Adsorption an Aktivkohle entfernt wird und gegebenenfalls anschließend ein mäßig wirksamer Polymerisationsinhibitor ausgewählt aus den Gruppe den phenolischen Verbindungen und Chimone zugegeben wird.

2. Verfahren nach Anspruch 1, **dadurch gekennzeichnet, dass** die Aktivkohle eine BET-Oberfläche von 200 bis 3000 m²/g aufweist.

3. Verfahren nach Anspruch 2, **dadurch gekennzeichnet, dass** die Aktivkohle eine BET-Oberfläche von 300 bis 2000 m²/g aufweist.

4. Verfahren nach einem der vorstehenden Ansprüche, **dadurch gekennzeichnet, dass** es sich bei der Aktivkohle um eine Formkohle, Kornkohle oder Pulverkohle handelt.

5. Verfahren nach einem der vorstehenden Ansprüche, **dadurch gekennzeichnet, dass** der Phenothiazin-Gehalt nach Adsorption unter 10 ppm beträgt.

6. Verfahren nach Anspruch 5, **dadurch gekennzeichnet, dass** der Phenothiazin-Gehalt nach Adsorption unter 5 ppm beträgt.

7. Verfahren nach einem der vorstehenden Ansprüche, **dadurch gekennzeichnet, dass** der mäßig wirksame Polymerisationsinhibitor ausgewählt ist aus der Gruppe der phenolischen Verbindungen und Chinone.

8. Verfahren nach Anspruch 7, **dadurch gekennzeichnet, dass** als mäßig wirksamer Polymerisationsinhibitor Hydrochinonomonomethylether verwendet wird.

9. Verfahren nach Anspruch 7 oder 8, **dadurch gekennzeichnet, dass** der Gehalt an mäßig wirksamem Polymerisationsinhibitor bis zu 100 ppm beträgt.

10. Verfahren nach einem der Ansprüche 7 bis 9, **dadurch gekennzeichnet, dass** der Gehalt an mäßig wirksamem Polymerisationsinhibitor um 50 ppm beträgt.

11. Verfahren nach einem der vorstehenden Ansprüche, **dadurch gekennzeichnet, dass** es sich bei dem (Meth)acrylmonomeren um (Meth)acrylsäure handelt.

12. Verfahren nach Anspruch 11, **dadurch gekennzeichnet, dass** es sich bei dem (Meth)acrylmonomeren um Acrylsäure für die Herstellung von superabsorbierenden Polymeren handelt.

13. Verfahren nach Anspruch 12, **dadurch gekennzeichnet, dass** die Acrylsäure einen Wassergehalt von 40 bis 75 Gew.-% aufweist.

14. Verfahren nach Anspruch 11, **dadurch gekennzeichnet, dass** die Acrylsäure wasserfrei ist.

15. Verfahren nach einem der Ansprüche 12 bis 14, **dadurch gekennzeichnet, dass** die Säuregruppen der Acrylsäure im Bereich von 20 bis 95 mol-% neutralisiert sind.

16. Verfahren nach einem der Ansprüche 12 bis 14, **dadurch gekennzeichnet, dass** die Säuregruppen der Acrylsäure unneutralisiert sind.

## Claims

1. A process for replacing the stabilization of (meth)acrylic monomers, which comprises removing phenothiazine from phenothiazine-containing (meth)acrylic monomers by adsorption on activated carbon and optionally subsequently adding a moderately active polymerization inhibitor selected from the group of the phenolic compounds and quinones.

2. The process according to claim 1, wherein the activated carbon has a BET surface area of 200 to 3000 m²/g.

3. The process according to claim 2, wherein the activated carbon has a BET surface area of 300 to 2000 m²/g.

4. The process according to any of the preceding claims, wherein the activated carbon is molded carbon, granular carbon or pulverized carbon.

5. The process according to any of the preceding claims, wherein the phenothiazine content after adsorption is less than 10 ppm.

6. The process according to claim 5, wherein the phenothiazine content after adsorption is less than 5 ppm.

7. The process according to any of the preceding claims, wherein the moderately active polymerization inhibitor is selected from the group of the phenolic compounds and quinones.

8. The process according to claim 7, wherein the moderately active polymerization inhibitor used is hydroquinone monomethyl ether.

9. The process according to claim 7 or 8, wherein the content of moderately active polymerization inhibitor is up to 100 ppm.

10. The process according to any of claims 7 to 9, wherein the content of moderately active polymerization inhibitor is around 50 ppm.

11. The process according to any of the preceding claims, wherein the (meth)acrylic monomer is (meth)acrylic acid.

12. The process according to claim 11, wherein the (meth)acrylic monomer is acrylic acid for the preparation of superabsorbent polymers.

13. The process according to claim 12, wherein the acrylic acid has a water content of 40 to 75% by weight.

14. The process according to claim 11, wherein the acrylic acid is anhydrous.

15. The process according to any of claims 12 to 14, wherein the acid groups of the acrylic acid are neutralized in the range from 20 to 95 mol%.

16. The process according to any of claims 12 to 14, wherein the acid groups of the acrylic acid are unneutralized.

## Revendications

1. Procédé pour la restabilisation de monomères de (méth)acryle, **caractérisé en ce qu'**on élimine par adsorption sur du charbon actif la phénothiazine de monomères de (méth)acryle contenant de la phénothiazine et on ajoute le cas échéant consécutivement un inhibiteur de polymérisation modérément actif choisi dans le groupe des composés phénoliques et des quinones.

2. Procédé selon la revendication 1, **caractérisé en ce que** le charbon actif présente une surface BET de 200 à 3000 m²/g.

3. Procédé selon la revendication 2, **caractérisé en ce que** le charbon actif présente une surface BET de 300 à 2000 m²/g.

4. Procédé selon l'une quelconque des revendications précédentes, **caractérisé en ce qu'**il s'agit, pour le charbon actif, d'un charbon façonné, d'un charbon en grains ou d'un charbon en poudre.

5. Procédé selon l'une quelconque des revendications précédentes, **caractérisé en ce que** la teneur en phénothiazine après l'adsorption est inférieure à 10 ppm.

6. Procédé selon la revendication 5, **caractérisé en ce que** la teneur en phénothiazine après l'adsorption est inférieure à 5 ppm.

7. Procédé selon l'une quelconque des revendications précédentes, **caractérisé en ce que** l'inhibiteur de polymérisation modérément actif est choisi dans le groupe des composés phénoliques et des quinones.

8. Procédé selon la revendication 7, **caractérisé en ce qu'**on utilise, comme inhibiteur de polymérisation modérément actif, l'hydrochinonomonométhyléther.

9. Procédé selon la revendication 7 ou 8, **caractérisé en ce que** la teneur en inhibiteur de polymérisation modérément actif est de jusqu'à 100 ppm.

10. Procédé selon l'une quelconque des revendications 7 à 9, **caractérisé en ce que** la teneur en inhibiteur de polymérisation modérément actif est d'environ 50 ppm.

11. Procédé selon l'une quelconque des revendications précédentes, **caractérisé en ce qu'**il s'agit, pour les monomères de (méth)acryle, d'acide (méth)acrylique.

12. Procédé selon la revendication 11, **caractérisé en ce qu'**il s'agit, pour les monomères de (méth)acryle, d'acide acrylique pour la préparation de polymères superabsorbants.

13. Procédé selon la revendication 12, **caractérisé en ce que** l'acide acrylique présente une teneur en eau de 40 à 75% en poids.

14. Procédé selon la revendication 11, **caractérisé en ce que** l'acide acrylique est anhydre.

15. Procédé selon l'une quelconque des revendications 12 à 14, **caractérisé en ce que** les groupes acides de l'acide acrylique sont neutralisés dans la plage de 20 à 95% en mole.

16. Procédé selon l'une quelconque des revendications 12 à 14, **caractérisé en ce que** les groupes acides de l'acide acrylique ne sont pas neutralisés.
